(19) **Europäisches Patentamt · European Patent Office · Office européen des brevets**

(11) **EP 0 971 227 B1**

(12) **EUROPEAN PATENT SPECIFICATION**

(45) Date of publication and mention of the grant of the patent:
**25.11.2009 Bulletin 2009/48**

(51) Int Cl.:
*G01N 22/04* (2006.01)

(21) Application number: **98109362.8**

(22) Date of filing: **22.05.1998**

(54) **Process for moisture measurement**

Verfahren zur Feuchtigkeitsmessung

Procédé pour la mesure de l'humidité

(84) Designated Contracting States:
**DE GB**

(43) Date of publication of application:
**12.01.2000 Bulletin 2000/02**

(73) Proprietor: **NATIONAL RESEARCH DEVELOPMENT CORPORATION**
**New Delhi 110 048 (IN)**

(72) Inventors:
- **Joshi,Kalpana,**
  **Sir Parshurambhau College**
  **Maharashtra (IN)**
- **Aiyer,Rohini,**
  **Dept.of Physics,University of Pune**
  **Maharashtra (IN)**
- **Karekar,Ravi Narhar,**
  **Dept.of Physics,Univ.of Pune**
  **Maharashtra (IN)**
- **Abegaonkar,Mahesh,**
  **Dept.of Physics,Univ.of Pune**
  **Maharashtra (IN)**

(74) Representative: **Raynor, Simon Mark et al**
**Urquhart-Dykes & Lord LLP**
**Midsummer House**
**413 Midsummer Boulevard**
**Central Milton Keynes MK9 3BN (GB)**

(56) References cited:
| | |
|---|---|
| GB-A- 2 277 803 | US-A- 4 408 128 |
| US-A- 4 991 915 | US-A- 5 313 167 |
| US-A- 5 334 941 | US-A- 5 420 517 |
| US-A- 5 646 537 | |

- DATABASE WPI Section EI, Week 8434 Derwent Publications Ltd., London, GB; Class S03, AN 84-211873 XP002083086 & SU 1 062 577 A (TRUBITSYNA O N) , 23 December 1983
- NELSON S O ET AL.: "MOISTURE DETERMINATION IN SINGLE GRAIN KERNELS AND NUTS BY RF IMPEDANCE MEASUREMENTS" IEEE TRANSACTIONS ON INSTRUMENTATION AND MEASUREMENT, vol. 41, no. 6, 1 December 1992, pages 1027-1031, XP000358690
- KRASZEWSKI ET AL.: "Moisture content determination in single peanut kernels with a microwave resonator." PEANUT SCIENCE, vol. 20, 1993, pages 27-31, XP002083085
- M. HAMID: "A Leaky Stripline Moisture Sensor" IEEE TRANSACTIONS ON INSTRUMENTATION AND MEASUREMENT, vol. 41, no. 4, August 1992 (1992-08), pages 560-562, New York, US
- JOSHI K K; ABEGAONKAR M; KAREKAR R N; AIYER R C: "Microstrip ring resonator as a moisture sensor for wheat grains" 1997 IEEE MTT-S INTERNATIONAL MICROWAVE SYMPOSIUM DIGEST (CAT. NO. 97 CH 36037), vol. 3, 1997, pages 1679-1682, New York, NY, USA ISBN: 0-7803-3814-6
- SARABANDI K; LI E S: "Characterization of soil moisture using a microstrip resonator" IGARSS '94. INTERNATIONAL GEOSCIENCE AND REMOTE SENSING SYMPOSIUM. SURFACE AND ATMOSPHERIC REMOTE SENSING: TECHNOLOGIES, DATA ANALYSIS AND INTERPRETATION (CAT. NO.94CH3378-7), vol. 3, 1994, pages 1445-1447, New York, NY, USA ISBN: 0-7803-1497-2

## Description

The present invention relates to a process for determining the content of moisture in grains. More precisely it relates to a process using a moisture sensing instrument which uses the measurement of dielectric properties for quantifying the moisture content of grains working in the range of microwave frequencies.

The process for measuring moisture content in grains uses a non-destructive moisture sensing instrument which uses dielectric properties and effective microstrip permittivity for measuring the moisture content of grains in Time Domain.

## BACKGROUND OF THE INVENTION

It is essential to determine the moisture content in the articles especially relating to articles used in food industry. The knowledge about the moisture content is essential factor for selecting the storage conditions and the safe storage period of a particular sample. Moisture content of various industrial materials like rubbers, foam, soap cakes, pharmaceutical products is an important factor for storage, shelf life and quality control.

Measurement of dielectric properties is important in the industrial processes involving polymers, rubbers, ceramics and plastics for the quality control as well as correct chemical compositions of the product in the liquid, semisolid or solid state.

Standard gravimetric laboratory tests are tedious and require several hours for completion, also they are destructive in nature. Number of conductivity based and capacitive techniques measure average moisture in bulk materials and need particular size of the sample to be measured. Moisture meters with penetrating probes are suitable for semisolid or grainy material or liquids only. Waveguide resonant cavity techniques have been widely used to perform non-destructive moisture measurements as well as complex permittivities of semiconductor materials. The waveguide cavity technique is associated with various difficulties like loading and unloading of samples even though it is considered as an accurate technique.

Moisture measurements in materials like rubber lining need a sensor which can measure moisture with surface contact only. The present technique can also be used for the measurement of thickness of ice formed on the wings of the aircraft of a space shuttle.

The moisture content of grains, seeds and food products such as wheat, rice, coffee is an important factor for the storage of grain, determination of the time of harvesting, marketing and processing (S.O. Nelson, V.K. Chari Kandala and Kurt C. Lowrence. "Moisture determination in single grain kernels and Nuts by RF Impedance Measurements". IEEE Trans. on Instrumentation and Measurements, Vol 41, No. 6, December 1992 and Andrezej W. Kraszevaski, "Microwave Aquametry-Needs and Perspective," IEEE Trans. on Microwave theory and techniques, Vol 39, No. 5, May 1991)

Moisture measurement is performed on the samples from yields of a cereal or pulse product to correct the yield figure. The error of moisture content of 1% in one ton of yields can cause an error of 10 Kg. Forecasting of the yields is important for enabling to take an action against the unbalanced demand and supply of the cereal products. It is extremely important to store the grain stock only after the moisture contents have been measured to ensure the prevention of insect manifestation. Measurement of moisture of materials is important in several industrial processes such as production of soaps. Similarly moisture measurement for liquids such as fruit products is essential. Measurement of moisture of materials is important in several industrial processes such as production of soaps, powders, biscuits, sugar syrups and for oil based products such as margarine.

The prior art cites references to different methods of qauntifying the moisture contents. Moisture meters/sensors have been used which include the standard gravimetric laboratory tests which are tedious and require several hours and days for completion. Conventional electric and electronic moisture meters measure average moisture in bulk grain sample. The average does not provide any information on the range of moisture in the individual grains or seed moisture content [A.W. Kraszevaski and S. O. Nelson, "Moisture content determination in Single Peanut Kernels With a Microwave resonator," Peanut Science, (1993) 20:27.31.]

U.S. Patent number 4, 408, 128 works on the principle that the electric capacity of a sample held between positive and negative electrodes is measured and the value of moisture content thereof is indicated on the basis of the correlation studies in advance between moisture content and electrical characteristic.

Due to the variation in the grain size, a single moisture meter fails to serve the purpose of all cereal products. It is therefore necessary to provide a moisture meter having correlation's calibrated for five respective cereal products according to the type of grain size. The apparent difference in the water content value caused by the difference in the temperature at the time of measurement makes correction by temperature desirable.

A method where a plurality of keys designating the type of sample with programmable EPROM read only memory capable of erasure and rewriting for storing parameters for computation are provided, is also known in the art. The EPROM is fed with the precalibrated values of capacitor voltage and moisture content. The indicator indicates the moisture content in the digital form. However, in this patent it is not clear whether the instrument measures moisture in a single grain or in a bulk of sample, e.g., 100gm of grains. The description of the actual sensor geometry and dimensions is not drawn or given. The drawbacks associated with this patent specifically relate to the fact that the single type of grain like wheat or rice has variation in the grain size. The capacity is a function of thickness and area and permittivity of the material between the plates. The said instrument does not give effective variation in the capacity with the size and weight of individual grains. If they measure 100g of grains, air gaps between the grains effect the reading thereby indicating a lower moisture content then the actual true value.

Another U.S. Patent 5646537 describes a time domain reflectometry waveguide assembly for measuring the moisture content in a medium and comprises calculating the apparent dielectric constant value of said medium based on a time delay measured in response to said detectable characteristic reference reflection, and correlating the apparent dielectric constant value with data reflecting the moisture content of the medium.

Another U.S. Patent 5420517 defines a time domain reflectometry waveguide assembly for measuring the moisture content in a medium.

Microwave moisture sensor (U.S. patent No. 499 1915, issued on 2.12.1991) works on the principle of absorption of the microwave energy into the material. This type of sensor is useful in case of high moisture content as the attenuation characteristics of microstrip are sensitive to the heavy losses in the material under test. These sensors are based on the power measurement and are totally dependent on the calibration of the materials on the custom made sensor and totally dependent on the dynamic performance.

U.S. patent number 5313167 issued in 1994 teaches coaxial line for introducing microwave energy with the detector coaxial line kept at a distance.

The waveguide cavity measurement technique is associated with the difficulties of loading and unloading of individual kernels and placing them accurately inside the cavity. The number of direct current conductivity and capacitance measurement based moisture sensors available in the market can handle bulk grain samples only where it is impossible to measure the range of moisture contents or dielectric parameters of the grains or materials. They are also affected by the air gaps in the grain samples and conductivity's of the grain covers as well as non-uniform shapes. The conductivity based moisture meters need constant pressure to be applied to the bulk under measurements for the validity of look up tables. Hence they are subject to the human errors.

Low frequency techniques are associated with the contributions due to the ionic conductivity's of the moist grain and hence are subjected to inherent errors. These type of sensors are based on the power measurement and are totally dependent on the calibration of the materials on the custom made sensor and totally dependent on the dynamic performance of the detector system and its readout system performance.

The sensors which measure moisture in the 100g of grain samples are incapable of measuring a range of moisture in the grain stock. Mixing of grains containing different moisture contents lead to the degradation of the complete stock. Hence it is important to measure moisture in individual kernels. The same applies to the moisture measurements before harvesting for the estimation of the national or regional yield to predict the national needs for import or export. Similarly moisture measurement of liquid samples such as sugar syrups, milk products, fruits such as grapes, apples, watermelon etc. require determination of precise moisture content.

Moisture measurement in industrial products like rubbers, foams, soaps is a commercially important technique for the quality control. Number of techniques in the market include conductivity, capacitance, frequency shift, time domain and nuclear techniques. Most of these techniques are destructive and demand machining of samples to peculiar geometry of insertion of probes into the material.

A stripline moisture sensor is disclosed in "A Leaky Stripline Moisture Sensor" by M. Hamid 8096, IEEE. Transactions on Instruments and Measurements 41 (1992) August, No. 4, New York, US.

A microstrip ring resonator is disclosed in "Microstrip Resonator As A Moisture Sensor For Wheat Grains" by K.K. Joshi et al, 1997 IEE MTT-S Digest.

The drawbacks of the conventional methods for determination of moisture content hinder the exact determination of the moisture content. The need has therefore been realised for a method and an instrument for determining the moisture content in various samples which overcomes the problems mentioned above. Time domain technique is a powerful measurement tool for the dielectric constant of the sample under test.

## SUMMARY OF THE INVENTION

The present invention relates to a process for moisture content determination in grains. The process of the present invention uses the measurement of dielectric properties for quantifying the moisture content of grains working in the range of microwave frequencies.

The present invention also relates to a process for determining moisture content in grains. The grains for which moisture content is to be measured could be grains such as wheat, rice, maize, millet. More precisely it relates to non-destructive moisture sensing process which uses dielectric properties and effective microstrip permittivity for measuring moisture content of grains in Time domain.

Thus the present invention aims to overcome the drawbacks in the existing sensors by providing an accurate, reliable and repeatable, density and volume independent state of art computer aided microwave process for the measurement of dielectric properties and hence moisture in grains.

## DETAILED DESCRIPTION OF THE PRESENT INVENTION

Measuring and monitoring moisture content helps in maintaining the quality and increasing the shelf life of a grain. Dielectric constant of pure water lies in the range of 75-80 at different frequencies. Dielectric constant of dry grain, have a much smaller value.

The novel process of the present invention is useful in measuring the moisture contents of various grains such as wheat, rice, maize, millet.

The object of the present invention is to provide a nondestructive microwave process for moisture measurement which obviates the drawbacks in the existing methods and provide accurate, reliable and repeatable results; which are density and volume independent.

An embodiment of the present invention resides in a process for the measurement of the moisture content of a grain, non destructively without physically probing material and by means of electromagnetic field probe in the vicinity of the stripline, microstripline or coplanar waveguide as an external probing instrument connected to a Time Domain reflectometer. A conventional Time Domain reflectometer is used for the measurement of delay in the reflection from the discontinuity in the characteristic impedance of the probing waveguide

In an embodiment of the present invention the microwave source may have frequencies above 1 Ghz in the microwave range.

In yet another embodiment of the invention the grain can be loaded on the sample holder without disturbing the precalibrated instrument.

In another feature of the present invention the response and recovery time of the sensor is of the order of one second.

The present invention offers nondestructive and relatively faster moisture measurement within grains of any shape or size.

The invention also defines a process and instrument for the measurement of effective permittivity of the microstrip, stripline or coplanar waveguide embedded with dielectric overlay or cover. The effective permittivity of any of the waveguide probes defined above is measured by means of measuring change in the delay, change in the reflection coefficient and/or change in the characteristic impedance due to the presence of sample as an overlay or dielectric cover over the microstrip, stripline or coplanar waveguide conducting probing instrument. The change in the effective permittivity is governed by the relation

$$t_s^2/t_o^2 = \varepsilon_{effs}/\varepsilon_{effo}$$

where suffices 'o' and 's' indicate time delay (t) and effective permittivity ($\varepsilon_{eff}$) without and with sample grain as an overlay.

The present time domain instrument is applicable for the measurement of moisture in grains of any size and shape with at least one dimension of the order or greater than 3mm. A microstrip and coplanar waveguide probe is used for the nondestructive measurement of moisture. The time domain reflectometer generating pulse inputs of picosecond resolution is connected to any one or multiple probes by means of a coaxial cable of matching impedance described above, The choice of the probe depends upon the geometry and the composition of the material. The grain under test is kept as an overlay on the microstrip probe. The change in the effective permittivity of the microstrip/coplanar waveguide probe is calibrated against moisture with optional electromagnetic software and a dedicated microcontroller or a computer. The calibration data is stored in the computer to give a directly correlated moisture percentage on the dedicated portable laptop or a fixed computer.

The object of the present invention is to provide non-destructive, accurate, fast, reliable and repeatable moisture measurement which eliminates the drawbacks of existing processes.

In an embodiment of the present invention the Time domain Reflectometer may have a pulse or step output containing frequency components between 0-20 Ghz or less with a coaxial cable carrying the step or pulse output of the Time Domain Reflectometer.

The change of the moisture content of wheat grain causes change in the effective permittivity and hence the resonant frequency of a microstrip resonator. The change in the complex permittivity causes change in the Q factor.

The resonant frequency of the microstrip resonator is highly sensitive to the presence of an overlay sample. Q factor decreases by about 48-53% when the moisture content increases by 30-41% whereas when the moisture content decreases by about 1-6% the Q factor increases by 10-20%. Therefore the Q factor and the moisture content have been known to be inversely proportional to each other. The lowest sensitivity has been observed in 0° orientation and 90° orientation gives the highest sensitivity.

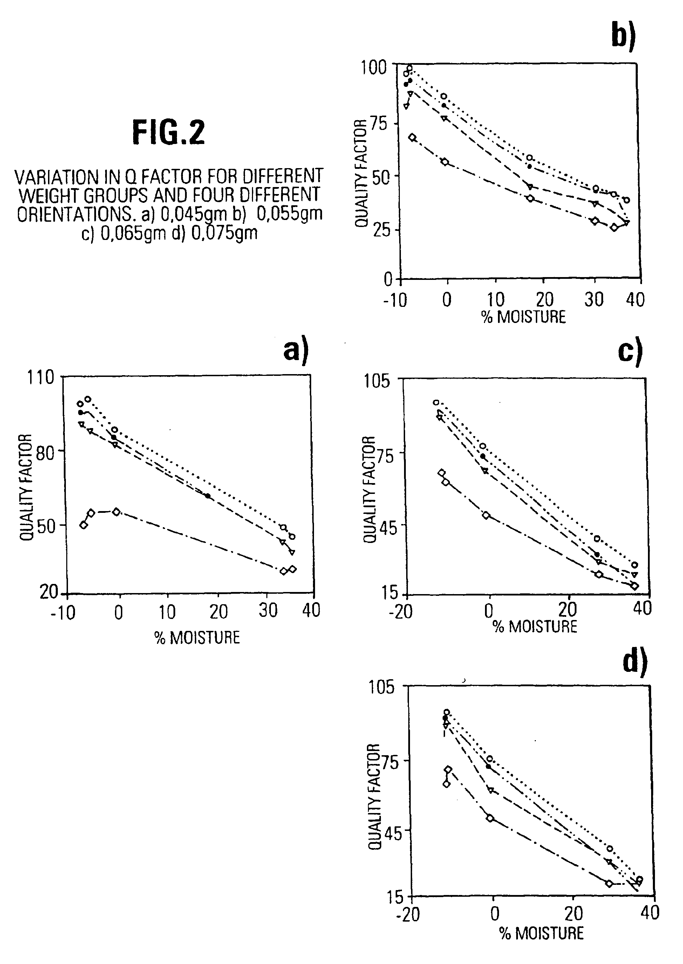

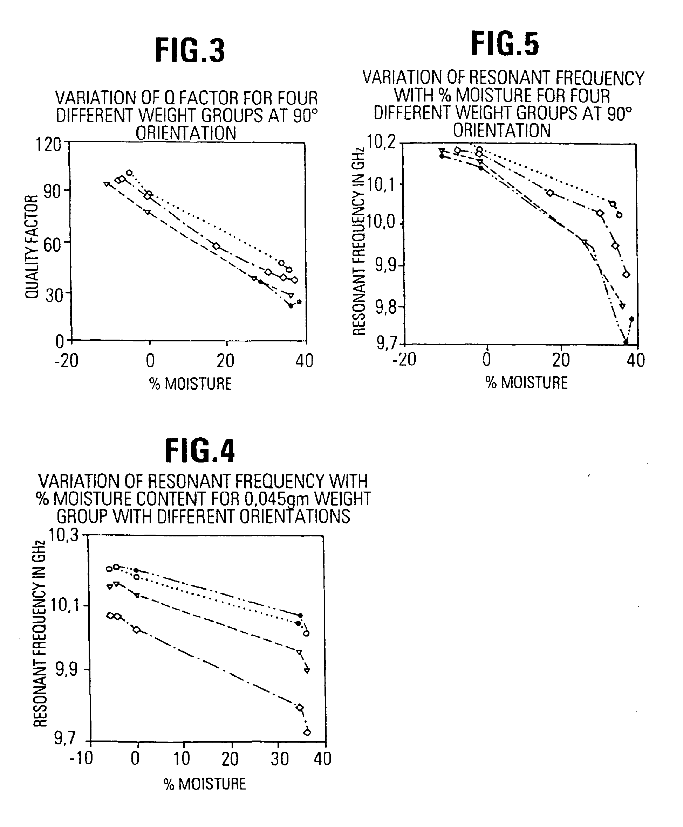

Figure 2 shows the variation in the Q factor for different weight groups and four different orientations. Figure 3 shows Q factor variation for four different weight groups at 90° orientation. Figure 4 shows the variation of resonant frequency with percentage moisture content for a particular weight group with different orientations. Figure 5 shows variation of resonant frequency with percentage moisture for four different weight groups at 90° orientation.

The results indicate that the Q factor measurements are weight independent as the Q factors lie in the same range even though the changes in the weight of the kernels are 45%. The sensitivity factor dQ/dM varies for different orientations where M indicates percentage moisture.

Regarding the time domain instrument the functional hybrid microwave transmission line resonator circuit consists of a microstrip, stripline or coplanar waveguide resonator could be a direct coupled either open ended or short circuited with a sample holder to hold sample in a particular position covering the entire area of the substrate and extending beyond the end of the conductor and substrate of a smaller size than that of the length of the resonator or a microstrip rectangular patch with a sample holder to hold sample under test in a particular position covering the entire area of the substrate and extending beyond the end of the conductor and substrate of a smaller size than that of the length of the patch.

According to the present invention the functional hybrid through microwave transmission line circuit consists of a microstrip, stripline or coplanar waveguide.

The functional hybrid through microwave transmission line on a resonator circuit consists of either microstrip, stripline or coplanar waveguide delineated on glass, ceramic, polymer or semiconductor substrate with chrome gold or copper gold conductor pattern with optional thin dielectric coating also with optional sample holding structure.

In another embodiment of the present invention a process and technique to measure the effective permittivity of a grain as an overlay without disturbing the calibration of the instrument without the grain hence avoiding error due to the connector repeatability is defined.

In another feature of the present invention the process and technique to measure the effective permittivity of a grain as an overlay has been defines where the response and recovery time of the sensor instrument is of the order of a fraction of a second.

The process for the measurement of the moisture content comprises of steps of calibrating the Time domain reflectometer, selecting probe dimensions and type, marking start of the probe by placing the marker on the Time Domain Reflectometer screen, placing the grain under test on the probe and analysing the data with the computer or look up tables and get moisture percentage.

The process for the measurement of moisture content of a grain using a time domain reflectometry assembly, comprises

calibrating a time domain reflectometer;

selecting microstripline, stripline or coplanar waveguide probe suitable for the type of sample;

connecting a coaxial cable to the probe;

positioning the grain on a sample holder;

transmitting a pulse signal of the kind used in time domain reflectometry through the coaxial cable;

commencing a measurement of time delay in response to the characteristic reference reflection;

calculating effective permittivity based on the time delay and

calibrating effective permittivity against moisture with optional electromagnetic software and a dedicated microcontroller or a computer or with calibration graphs and tables.

The time domain reflectometer as already mentioned generates the pulse of picosecond duration. The said sample is placed on a microstrip patch covering the entire area of the substrate and extending beyond the end of the conductor and substrate or substrate of smaller size than that of the length of the said resonator.

The instrument used in the present invention optionally comprises spacer block placed over the said grain for putting pressure on the grain. This spacer block therefore enables removing the air gap present. The presence of air gap causes an error in the measurement of moisture content which could vary the results by as big as 10% error. Therefore spacer blocks of effective permittivity of approximately 1 can be defined.

A probe length may be varied depending upon the grain, dielectric constant of the grain and moisture levels to be measured.

In another optional feature of the present feature of the present invention the microcontroller or a dedicated computer calculates effective permittivity and the moisture content of the grain under test or calibration graphs and tables provide correlation between the measured data on time delay for pulse step or step input signal to return or reflection coefficient or characteristic impedance of the sensor probe.

According to the present invention therefore there is provided a functional microwave functional hybrid microstrip or stripline or coplanar waveguide circuit, a voltage step generator and an instrument sampling the returned pulse. The presence of sample causes a change in the effective permittivity of the microstrip, stripline or a coplanar waveguide structure. The change in effective permittivity is therefore calibrated and the dielectric constant evaluated. To understand the present invention clearly a set of drawings are submitted wherein figure 1 shows the arrangement of the components of the instrument for measuring moisture content of the sample wherein the microwave source and functional microstrip sensor circuit and detector are arranged in a particular manner. Figure 2 shows the variation in the Q factor for different weight groups and four orientations while figure 3 represents Q factor variation for four different weight groups at 90° orientation. Figure 4 shows the variation of resonant frequency with percentage moisture content for a particular weight group with different orientations and figure 5 shows variation of resonant frequency with percentage moisture for four different weight groups at 90° orientation. Figure 6 shows the ring resonator with material under test.

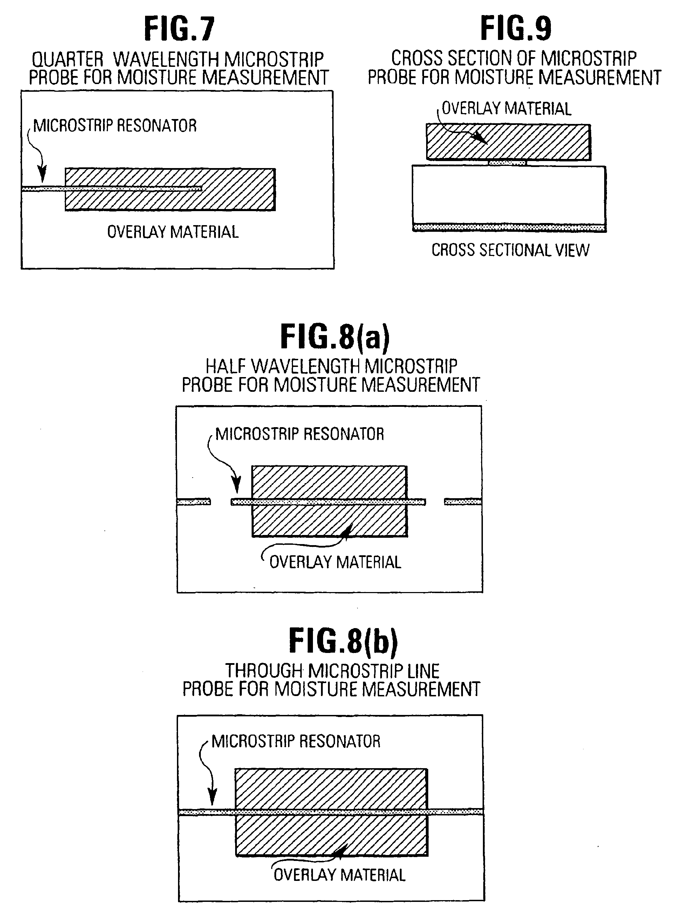

Figure 7 shows quarter wavelength microstrip probe for moisture measurement.

Figure 8(a) depicts half wavelength microstrip probe for moisture measurement and

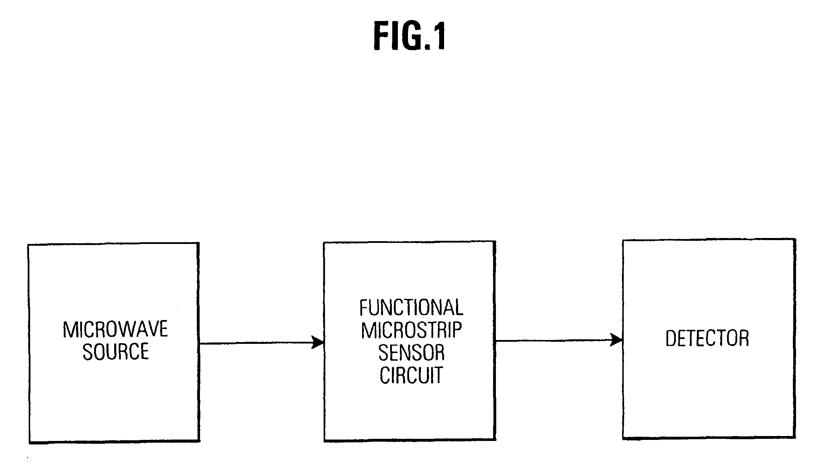

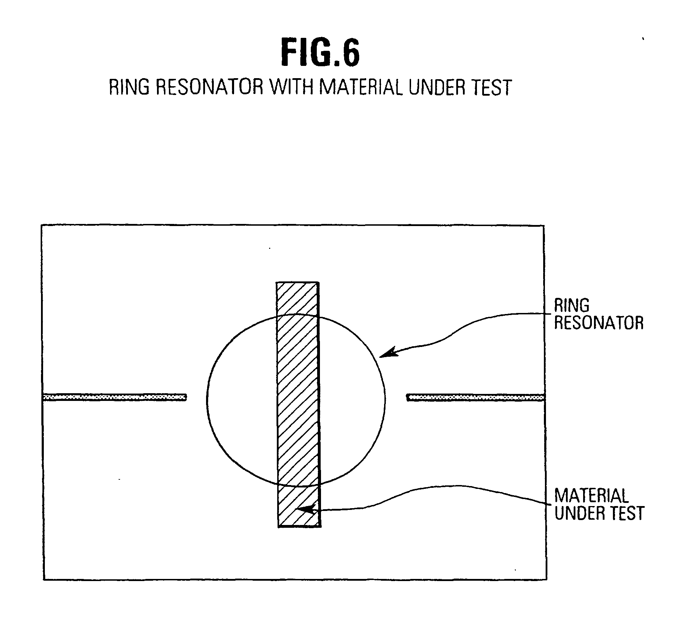

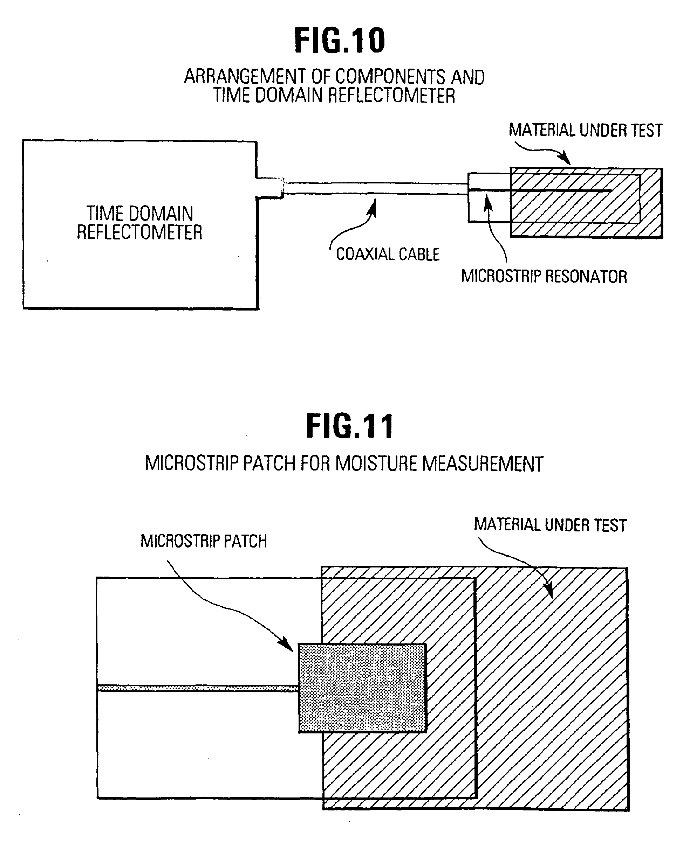

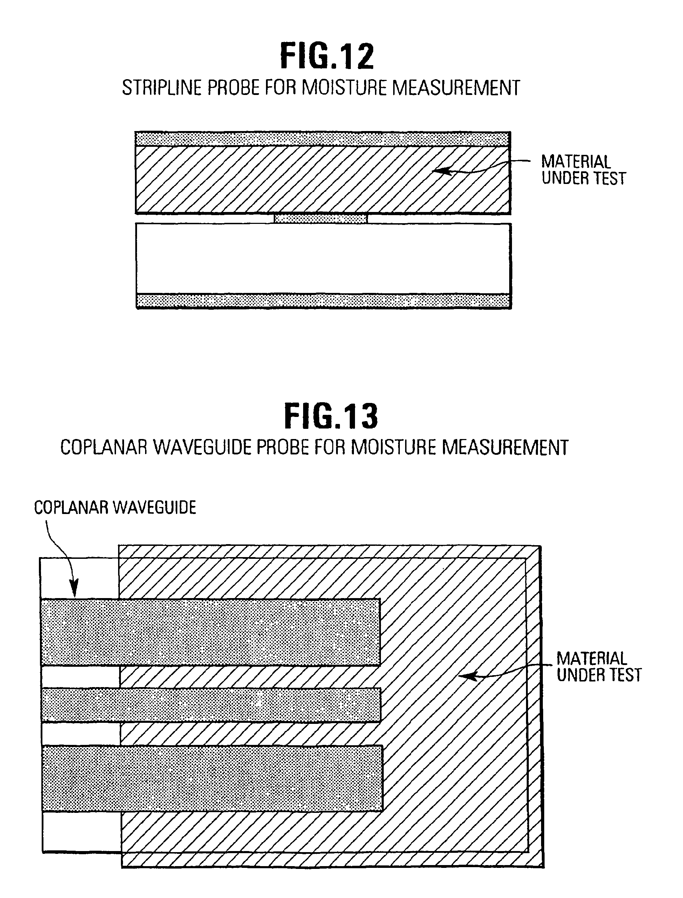

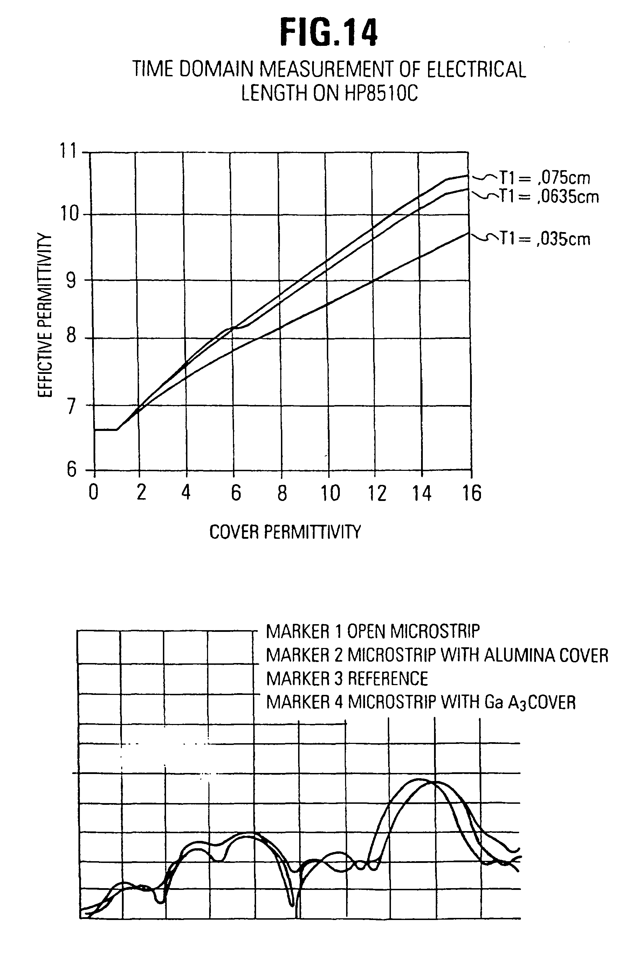

figure 8(b) shows a microstrip line probe for moisture measurement. Figure 9 shows cross section of microstrip probe for moisture measurement while figure 10 shows arrangement of components and the time domain reflectometer. Figure 11 shows microstrip patch for moisture measurement and figure 12 depicts stripline probe for moisture measurement. Figure 13 shows coplanar waveguide probe for moisture measurement. Figure 14 shows time domain measurement of electrical length on HP8510C. Figures 1 to 9 do not constitute embodiments of the invention.

The specification concludes with claims particularly pointing out and distinctly claiming the present invention. While the following examples do not constitute embodiments of the invention, it is believed the present invention will be better understood from the following description in conjunction with the accompanying drawings.

## EXAMPLES

About 100 wheat grains of a high quality "Lokawan" variety grown in Gujarat state of India are taken. These are classified into four weight groups:

(a) $0.045 \pm 0.005$ gm
(b) $0.055 \pm 0.005$ gm
(c) $0.065 \pm 0.005$ gm
(d) $0.075 \pm 0.005$ gm

The individual grains are kept on a ring at different orientations viz. 0°, 45° -45° and 90°. The change in the resonant frequency and change in the quality factor of the ring are recorded while varying the moisture levels from 15% to 40% by oven drying and soaking in water while considering the starting level as reference level. Q factor of the lowest weight group viz $\pm 0.045$ decreases by 51% when the moisture content of the grain is increases by 36% whereas as the moisture content decreases by 4% the Q factor increases by 15%. The lowest sensitivity is observed at 0° orientation and 90° orientation gives the highest sensitivity. The results are shown in the figures 2-5.

The results clearly indicate that the Q factor measurements are weight independent as the Q factor lies in the same range even though the changes in the weight of the kernels are 45%.

## Claims

1. A process for the measurement of moisture content of single grains, using an instrument comprising:

   a sample holder having the grain placed on it;
   a pulse generating source generating pulse inputs of picosecond resolution said pulse generating input source being a time domain reflectometer;
   a probe including a substrate and a microstrip, stripline or coplanar wave guide conductor for measurement of moisture contents connected to the said pulse generating source through a coaxial cable;
   means for calibrating the effective permittivity measured against the moisture content of the grain, the said means being a microcontroller or a computer or calibrated graphs
   the sample holder covering the entire width of the substrate and extending beyond the end of the conductor and substrate and covering less than the entire length of said probe;
   the process comprising
   calibrating a time domain reflectometer;
   selecting microstripline, stripline or coplanar waveguide probe suitable for the type of grain;
   connecting a coaxial cable to the probe;
   positioning the grain on the sample holder at a selected orientation;
   transmitting a pulse signal of the kind used in time domain reflectometry through the coaxial cable;
   commencing a measurement of time delay in response to the characteristic reference reflection;
   calculating effective permittivity based on the time delay and
   calibrating effective permittivity against moisture with said calibrating means.

2. A process as claimed in claim 1, comprising placing a spacer block over the grain for putting pressure on the grain.

3. A process as claimed in claim 2 wherein the length of said probe may be varied depending upon the grain dielectric constant of the grain and moisture levels to be measured

4. A process as claimed in claim 3, wherein the substrate is a glass, ceramic or polymer or semiconductor substrate, preferably comprising a copper gold conductor pattern with optional thin dielectric coating.

## Patentansprüche

1. Prozess zum Messen des Feuchtigkeitsgehalts einzelner Körner unter Benutzung eines Instruments, umfassend:

   einen Probenträger mit dem darauf angeordneten Korn;
   eine impulserzeugende Quelle, die Impulszufuhren mit Picosekundenauflösung erzeugt, wobei die impulserzeugende Zufuhrquelle ein Zeitbereichsreflektometer ist;
   einen Messfühler mit einem Substrat und einem Mikrostreifen, einer Streifenleitung oder einem koplanaren Wellenleiter zum Messen des Feuchtigkeitsgehalts, der über ein Koaxialkabel an die impulserzeugende Quelle angeschlossen ist;
   Mittel zum Kalibrieren der in Abhängigkeit des Feuchtigkeitsgehalts des Korns gemessenen effektiven Permittivität, wobei das Mittel ein Mikrocontroller oder ein Rechner oder kalibrierte Graphen ist;

wobei der Probenträger die gesamte Breite des substrats abdeckt und über das Ende des Leiters und Substrats hinaus verläuft und weniger als die gesamte Länge des Messfühlers abdeckt;

wobei der Prozess Folgendes umfasst:

Kalibrieren eines Zeitbereichsreflektometers;

Auswählen von Mikrostreifenleitungs-, Streifenleitungs- oder koplanarem Wellenleitermessfühler, der für die Kornart geeignet ist;

Abschließen eines Koaxialkabels an den Messfühler;

Anordnen des Korns auf dem Probenträger in einer gewählten Ausrichtung;

Übertragen eines Impulssignals der Art, die bei der Zeitbereichsreflektometrie benutzt wird, über das Koaxialkabel;

Einleiten einer Messung von Totzeit in Reaktion auf die charakteristische Bezugsreflexion;

Berechnen effektiver Permittivität auf Grundlage der Totzeit und

Kalibrieren effektiver Permittivität in Abhängigkeit von Feuchtigkeit mit dem Kalibriermittel.

**2.** Prozess nach Anspruch 1, umfassend das Anordnen eines Distanzblocks über dem Korn zum Ausüben von Druck auf das Korn.

**3.** Prozess nach Anspruch 2, wobei die Länge des Messfühlers abhängig von dem Korn, der Dielektrizitätskonstante des Korns und Feuchtegraden, die gemessen werden sollen, verändert sein kann.

**4.** Prozess nach Anspruch 3, wobei das Substrat ein Glas-, Keramik- oder Polymer- oder Halbleitersubstrat ist, das vorzugsweise ein Kupfer-Gold-Leiterbild mit optionaler dünner dielektrischer Beschichtung umfasst.

## Revendications

**1.** Procédé de mesure de la teneur en eau de grains isolés à l'aide d'un instrument, comprenant :

un support d'échantillon sur lequel est placé le grain ;

une source de génération d'impulsions qui génère des entrées d'impulsions qui présentent une résolution de la picoseconde, ladite source d'entrée de génération d'impulsions étant un réflectomètre de domaine temporel ;

une sonde qui comprend un substrat et un conducteur à guide d'ondes à microrubans, à rubans ou coplanaire pour la mesure des teneurs en eau, connectée à ladite source de génération d'impulsions par l'intermédiaire d'un câble coaxial ;

des moyens destinés à étalonner la constante diélectrique effective mesurée en fonction de la teneur en eau du grain, lesdits moyens étant un microcontrôleur ou un ordinateur ou des courbes étalonnées ;

le support d'échantillon couvrant toute la largeur du substrat et s'étendant au-delà de l'extrémité du conducteur et du substrat et couvrant moins que toute la longueur de ladite sonde ;

le procédé comprenant les étapes consistant à :

étalonner un réflectomètre de domaine temporel ;

sélectionner une sonde à guide d'ondes à microrubans, à rubans ou coplanaire, appropriée pour le type de grain ;

connecter un câble coaxial à la sonde ;

positionner le grain sur le support d'échantillon selon une orientation sélectionnée,

transmettre un signal d'impulsion de la sorte utilisée en réflectométrie de domaine temporel par l'intermédiaire du câble coaxial;

commencer une mesure de temps de retard en réponse à la réflexion de référence caractéristique ;

calculer la constante diélectrique effective sur la base du temps de retard ; et

étalonner la constante diélectrique effective en fonction de l'humidité à l'aide desdits moyens d'étalonnage.

**2.** Procédé selon la revendication 1, comprenant une étape consistant à placer un bloc espaceur au-dessus du grain de manière à exercer une pression sur le grain.

**3.** Procédé selon la revendication 2, dans lequel la longueur de ladite sonde peut varier selon la constante diélectrique du grain et les niveaux d'humidité à mesurer.

**4.** Procédé selon la revendication 3, dans lequel le substrat est un verre, une céramique ou un polymère ou un substrat semi-conducteur, qui comprend de préférence un motif conducteur de cuivre or avec éventuellement un revêtement diélectrique mince.

# FIG.1

MICROWAVE SOURCE → FUNCTIONAL MICROSTRIP SENSOR CIRCUIT → DETECTOR

EP 0 971 227 B1

**FIG.2**

VARIATION IN Q FACTOR FOR DIFFERENT
WEIGHT GROUPS AND FOUR DIFFERENT
ORIENTATIONS. a) 0,045gm b) 0,055gm
c) 0,065gm d) 0,075gm

# FIG.3

VARIATION OF Q FACTOR FOR FOUR
DIFFERENT WEIGHT GROUPS AT 90°
ORIENTATION

# FIG.5

VARIATION OF RESONANT FREQUENCY
WITH % MOISTURE FOR FOUR
DIFFERENT WEIGHT GROUPS AT 90°
ORIENTATION

# FIG.4

VARIATION OF RESONANT FREQUENCY WITH
% MOISTURE CONTENT FOR 0,045gm WEIGHT
GROUP WITH DIFFERENT ORIENTATIONS

# FIG.6
### RING RESONATOR WITH MATERIAL UNDER TEST

RING
RESONATOR

MATERIAL
UNDER TEST

# FIG.7

### QUARTER WAVELENGTH MICROSTRIP PROBE FOR MOISTURE MEASUREMENT

MICROSTRIP RESONATOR

OVERLAY MATERIAL

# FIG.9

### CROSS SECTION OF MICROSTRIP PROBE FOR MOISTURE MEASUREMENT

OVERLAY MATERIAL

CROSS SECTIONAL VIEW

# FIG.8(a)

### HALF WAVELENGTH MICROSTRIP PROBE FOR MOISTURE MEASUREMENT

MICROSTRIP RESONATOR

OVERLAY MATERIAL

# FIG.8(b)

### THROUGH MICROSTRIP LINE PROBE FOR MOISTURE MEASUREMENT

MICROSTRIP RESONATOR

OVERLAY MATERIAL

# FIG.10

## ARRANGEMENT OF COMPONENTS AND TIME DOMAIN REFLECTOMETER

MATERIAL UNDER TEST

TIME DOMAIN REFLECTOMETER

COAXIAL CABLE

MICROSTRIP RESONATOR

# FIG.11

## MICROSTRIP PATCH FOR MOISTURE MEASUREMENT

MATERIAL UNDER TEST

MICROSTRIP PATCH

# FIG.12

## STRIPLINE PROBE FOR MOISTURE MEASUREMENT

MATERIAL
UNDER TEST

# FIG.13

## COPLANAR WAVEGUIDE PROBE FOR MOISTURE MEASUREMENT

COPLANAR WAVEGUIDE

MATERIAL
UNDER TEST

# FIG.14

## TIME DOMAIN MEASUREMENT OF ELECTRICAL LENGTH ON HP8510C

MARKER 1 OPEN MICROSTRIP
MARKER 2 MICROSTRIP WITH ALUMINA COVER
MARKER 3 REFERENCE
MARKER 4 MICROSTRIP WITH Ga $A_3$COVER

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4408128 A
- US 5646537 A
- US 5420517 A
- US 4991915 A
- US 5313167 A

**Non-patent literature cited in the description**

- **S.O. Nelson ; V.K. Chari Kandala ; Kurt C. Lowrence.** Moisture determination in single grain kernels and Nuts by RF Impedance Measurements. *IEEE Trans. on Instrumentation and Measurements,* December 1992, vol. 41 (6
- **Andrezej W. Kraszevaski.** Microwave Aquametry-Needs and Perspective. *IEEE Trans. on Microwave theory and techniques,* May 1991, vol. 39 (5
- **A.W. Kraszevaski ; S. O. Nelson.** Moisture content determination in Single Peanut Kernels With a Microwave resonator. *Peanut Science,* 1993, vol. 20, 27-31
- **M. Hamid.** A Leaky Stripline Moisture Sensor. *IEEE. Transactions on Instruments and Measurements,* August 1992, vol. 41 (4
- **K.K. Joshi et al.** Microstrip Resonator As A Moisture Sensor For Wheat Grains. *IEE MTT-S Digest,* 1997